## Europäisches Patentamt

## European Patent Office

## Office européen des brevets

(19)

(11) Veröffentlichungsnummer: **0 076 444 B1**

(12) **EUROPÄISCHE PATENTSCHRIFT**

(45) Veröffentlichungstag der Patentschrift: 13.05.87

(51) Int. Cl.⁴: **A 61 K 7/06**

(21) Anmeldenummer: **82108889.5**

(22) Anmeldetag: **25.09.82**

(54) **Verwendung kolloidaler Lösungen von Seidenfibroin in haarkosmetischen Mitteln und Haarschampoo.**

(30) Priorität: **03.10.81 DE 3139438**

(43) Veröffentlichungstag der Anmeldung: **13.04.83 Patentblatt 83/15**

(45) Bekanntmachung des Hinweises auf die Patenterteilung: **13.05.87 Patentblatt 87/20**

(84) Benannte Vertragsstaaten: **AT BE CH DE FR GB IT LI LU NL SE**

(56) Entgegenhaltungen:
**DE - A - 2 151 740**
**FR - A - 1 501 006**
**FR - A - 2 210 380**

**CHEMICAL ABSTRACTS, Band 47, 1953, Spalte 5642b, Columbus, Ohio, USA - & JP - A - 52299**
**MONATSHEFTE FÜR CHEMIE, Band 85, Nr. 1, 1954, Seiten 120-139 - E. SCHAUENSTEIN et al.: "Ultraviolett-Absorptionsspektren von Seisenfibroin und Cellulose in Lithiumbromidlösung"**
**R. SIGNER und R. STRAESSLE, Helv. Chim. Acta. 30, 155-162 (1947)**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Ritter, Wolfgang, Dr., Oppelnerweg 3, D-4000 Düsseldorf (DE)**
Erfinder: **Giede, Karl, Schlehenweg 12, D-4010 Hilden (DE)**
Erfinder: **Höffkes, Horst, Dr., Stettiner Strasse 102, D-4000 Düsseldorf (DE)**

## Beschreibung

Gegenstand der Erfindung ist die Verwendung von kolloidalen Lösungen von Seidenfibroin in konzentrierten wässrigen Lösungen bestimmter Elektrolyte als haarpflegende Zusätze zu haarkosmetischen Mitteln. Haarkosmetische Mittel im Sinne dieser Erfindung sind Zusammensetzungen, die zum Waschen, Nachspülen, Pflegen, Verformen, Bleichen, Färben, Festigen oder Frisieren des Haares bestimmt sind. Ein weiterer Gegenstand der Erfindung ist ein Haarshampoo, welches einen Zusatz an kolloidal gelöstem Seidenfibroin enthält.

Es ist bekannt, kosmetischen Präparaten, insbesondere haarkosmetischen Mitteln, modifizierte Proteine zur Erhöhung der pflegenden Eigenschaften zuzusetzen. Zu diesem Zweck werden entweder wasserlösliche Proteine verwendet oder die Proteine müssen in eine wasserlösliche Form überführt werden. Die nicht wasserlöslichen Proteine werden üblicherweise durch chemischen oder enzymatischen Abbau, also unter weitgehender Hydrolyse der Peptidbindungen in eine wasserlösliche Form überführt. Obwohl es zur Steigerung der haarkosmetischen Wirkung wünschenswert wäre, die Polypeptid-Struktur nicht oder nur wenig abzubauen, ist zur Erzielung einer ausreichenden Wasserlöslichkeit bei Faserproteinen ein so weitgehender Abbau erforderlich, dass die kosmetische Wirksamkeit, die auf das substantive Aufziehvermögen auf Haare und Haut zurückgeführt wird, nicht mehr befriedigt.

Es ist auch bekannt, Proteine durch Umsetzung mit 2 bis 10 Kohlenstoffatome enthaltenden Di- und/oder Polyaminen abzubauen und die erhaltenden Protein-Aminolysate weiter mit Epoxiden und Fettsäureestern umzusetzen. Die auf diese Weise nach der deutschen Patentschrift 2 151 740 erhaltenen Protein-Derivate zeigen gegenüber einfachen Protein-Hydrolysaten und Aminolysaten gewisse anwendungstechnische Vorzüge, befriedigen jedoch nicht das Bedürfnis nach einem möglichst naturbelassenen, also die natürliche Struktur noch aufweisenden kosmetischen Wirkstoff, der trotzdem eine hohe haarkosmetische Wirksamkeit entfaltet. Dieses Bedürfnis kann erst recht durch synthetische, zum Beispiel kationische Polymere nicht befriedigt werden.

Es bestand also die Aufgabe, die haarkosmetischen Eigenschaften, insbesondere die haarpflegenden Eigenschaften von haarkosmetischen Mitteln durch den Zusatz eines nicht oder möglichst wenig abgebauten und chemisch nicht modifizierten Faserproteins zu verbessern.

Aus Chem. Abstr. 1953, Bd. 47, 5642b und JP-A-52 299 war die Verwendung einer Fibroinzubereitung, die durch Kochen einer entbasteten Seide in CaCl$_2$-Lösung und Entfernung des Elektrolyten durch Ionenaustausch erhalten wurde, als kosmetisches Rohmaterial bekannt. Das Fibroin wird dabei offenbar so weit chemisch abgebaut, dass es nach Entfernung des Elektrolyten gelöst bleibt. Aus Monatshefte für Chemie, Bd. 85, Nr. 1 (1954), Seiten 120 bis 139 (Schauenstein et al) war bekannt, dass sich Seidenfibroin in konzentrierter LiBr-Lösung auflösen lässt. Auch aus Helv. Chim. Acta. 30, Seite 155 ff (R. Singer und R. Straessle) war bekannt, dass sich Seidenfibroin durch Einwirkung von Lithiumjodid, -bromid und -rhodanid in kolloidale Lösungen überführen lässt. In FR-A-1 501 006 wird ein durch Mahlung erhältliches Seidenpulver als Bestandteil kosmetischer Produkte beschrieben.

Aus Veröffentlichungen von P.P.v. Weimarn in Kolloid-Zeitschrift, Band 40 (1926), S. 120–122 und Band 41 (1926), S. 143–152 ist bekannt, dass sich wasserunlösliche Polysaccharide wie Chitin und Cellulose sowie unlösliche Polypeptide wie Fibroin und Keratin in konzentrierten Lösungen bestimmter Salze auflösen lassen. Die Auflösung erfolgt bei Wahl geeigneter Salze unter sehr schonenden Bedingungen, bei niedriger Temperatur und bei neutralem pH-Wert. Die erhaltenen kolloidalen Lösungen zeichnen sich durch hohe Viskosität aus und ermöglichen durch Verspinnen in ein Koagulationsbad die Gewinnung von Fasern, was auf einen sehr geringen chemischen Abbau schliessen lässt. Während in den zitierten Arbeiten eine mögliche Eignung solcher Lösungen zur Erzeugung regenerierter Fasern untersucht wurde, ist ein Hinweis auf eine kosmetische Brauchbarkeit nicht zu entnehmen.

Es wurde nun gefunden, dass kolloidale Lösungen von Seidenfibroin in konzentrierten wässrigen Lösungen der Chloride und/oder Bromide des Lithiums und/oder Magnesiums sich überraschend gut zur Verwendung als haarpflegende und avivierende Zusätze zu haarkosmetischen Mitteln eignen. Als haarkosmetische Mittel im Sinne der Erfindung sind Zubereitungen zum Waschen, Nachspülen, Pflegen, Verformen, Bleichen, Färben, Festigen und Frisieren des Haares zu verstehen. Die erfindungsgemässe Verwendung der kolloidalen Lösungen von Seidenfibroin erstreckt sich daher zum Beispiel auf den Zusatz zu Haarshampoos, Haarnachspülmitteln, Haarkuren, Haarwellmitteln, Haarbleichmitteln, Haarfärbemitteln, Haarfestigern und Frisierhilfsmitteln.

Den genannten haarkosmetischen Zusammensetzungen verleiht der Zusatz an kolloidal gelöstem Fibroin vorteilhafte Anwendungseigenschaften. Während die erfindungsgemässe Verwendung der kolloidalen Lösungen des Seidenfibroins in Haarshampoos und Haarnachspülmitteln vor allem zu einer Erhöhung von Glanz und Fülle sowie zu einer Verbesserung der Kämmbarkeit des behandelten Haares führt, wird in Haarfestigern, in Haarfönlotionen und Haarfrisierhilfsmitteln die Elastizität und der Sitz des Haares günstig beeinflusst. In Haarwellmitteln und Haarfärbe- sowie Haarbleichmitteln wird durch die erfindungsgemässe Verwendung der kolloidalen Lösungen von Seidenfibroin darüber hinaus eine Verringerung der haarschädigenden Wirkung dieser Haarbehandlungsmittel und eine weitgehende Erhaltung von Glanz und Elastizität des Haares erreicht. Besonders ausgeprägt sind die beschriebenen kosmetischen Effekte, wenn kolloidale Lösungen von Seidenfibroin verwendet werden, die durch Lösung des Seidenfibroins bei Temperaturen unterhalb +60°C in einer konzentrierten wässrigen Lösung von Lithiumbromid erhalten werden.

Die Herstellung der kolloidalen Lösung des Seidenfibroins ausgehend von Naturseide erfordert zunächst ein Abtrennen der die Seidenfaser umhüllenden, aus dem leimartigen Protein Sericin bestehenden Schutzschicht. Dieses Entbasten der Seide ist z.B. durch Behandlung mit kochendem Wasser nach L.M. Kay und W.A. Schroeder, J. Am Chem. Soc. 76, (1964), Seite 3546, mit kochender, wässriger Seife nach R. Signer und R. Strässle, Helv. Chim. Acta 30, (1947), Seite 155 oder mit verdünntem, wässrigem Alkali, wässrigem Natriumcarbonat oder proteolytischen Enzymen durchzuführen.

Die entbastete Seide wird in konzentrierte wässrige Lösungen von Lithiumchlorid, Lithiumbromid, Magnesiumchlorid, Magnesiumbromid oder von Mischungen dieser Salze eingetragen, die Auflösung des Seidenfibroins erfolgt bei niedrigen Temperaturen langsam im Verlaufe von einigen Stunden bis zu mehreren Tagen. Durch Erwärmen der Salzlösung kann der Lösevorgang erheblich beschleunigt werden. Als konzentrierte Lösungen der genannten Salze werden wässrige Lösungen verstanden, die mehr als 1/3, vorzugsweise mehr als 2/3 der bei 20°C zur Sättigung der Lösung erforderlichen Menge dieser Salze enthalten. Es ist vorteilhaft, eine möglichst hohe Konzentration des kolloidalen Seidenfibroins in der konzentrierten Salzlösung zu verwenden, damit nicht mehr Salz als zur Lösung des Seidenfibroins erforderlich, in die haarkosmetischen Mittel gelangt. Andererseits ist der erreichbaren Konzentration des Seidenfibroins in der konzentrierten Salzlösung durch Art und Konzentration des verwendeten Salzes, Lösetemperatur und Lösezeit sowie durch die sich ergebende hohe Viskosität der kolloidalen Lösung eine obere Grenze gesetzt. Besonders vorteilhaft zu verarbeiten sind Lösungen von 8 bis 12 Gewichtsprozent Seidenfibroin in wässrigen Lösungen von 40–60 Gewichtsprozent Lithiumbromid.

Die kolloidalen Lösungen des Seidenfibroins lassen sich in üblicher Weise durch einfaches Einrühren in die verschiedenen haarkosmetischen Mittel einarbeiten. Zu Ausfällungen und Unverträglichkeiten mit den Bestandteilen der Formulierungen kommt es gewöhnlich nicht. Austrübungen können bei zu hoher Dosierung und in Rezepturen mit hohem Gehalt an niederen Alkoholen auftreten. Zu Formulierungen mit anionischen Sulfat- und Sulfonat-Tensiden, wie z.B. in Haarwaschmitteln, kann durch die Mitverwendung von Lithiumsalzen dieser Aniontenside eine Erhöhung der Lagerstabilität erreicht werden.

Bevorzugt ist die Verwendung der kolloidalen Lösungen des Seidenfibroins als Zusatz zu den haarkosmetischen Formulierungen in Mengen, die einer kolloidal gelösten Menge von 0,01 bis 1,0 Gewichtsprozent Seidenfibroin entsprechen.

Als besonders vorteilhafte Ausführung der Erfindung hat sich ein Haarshampoo erwiesen. Dieses enthält erfindungsgemäss 0,1 bis 1,0 Gewichtsprozent kolloidal gelöster Seide, die zur Auflösung der Seide erforderliche Menge an Chloriden und/oder Bromiden des Lithiums und/oder Magnesiums, 5 bis 20 Gewichtsprozent anionischer Sulfattenside wie Fettalkoholsulfate und Fettalkoholpolyglykolethersulfate mit 2 bis 10 Glykolethergruppen oder Sulfonattenside wie sekundäre Alkansulfonate mit 10 bis 20 Kohlenstoffatomen und α-Oleinsulfonate mit 10 bis 20 Kohlenstoffatomen, in Form der Ammonium-, Alkanolammonium, Alkali- oder Magnesiumsalze sowie gegebenenfalls nichtionische und ampholytische Tenside, Fettsäurealkanolamide und weitere übliche Shampoobestandteile. Besonders vorteilhaft ist der zumindest teilweise Einsatz der anionischen Sulfat- und Sulfonattenside in Form der Lithiumsalze, da sich durch diese Massnahme grössere Mengen an kolloidalen Lösungen des Seidenfibroins ohne Auftreten von Austrübungen einarbeiten lassen als bei Verwendung der Natriumsalze.

Die erfindungsgemässen Haarshampoos können auch nichtionogene Waschrohstoffe enthalten, wie z.B. Fettalkoholpolyglykolether mit 6 bis 12 Glykolethergruppen, Alkylphenolpolyglykolether mit 8 bis 12 Kohlenstoffatomen in der Alkylgruppe und 6 bis 15 Glykolethergruppen im Molekül, Fettsäureamid-Polyglykolether mit 3 bis 6 Polyglykolethergruppen. Die erfindungsgemässen Haarshampoos können auch ampholytische Tenside wie z.B. Dimethylfettaminoessigsäure-Betain, Fettacylamidopropyldimethylaminoessigsäure-Betain, Aminoxide tertiärer Fettamine oder Fettacylamidoalkylamine, ferner Imidazolinium-Betaine oder Sulfobetaine enthalten. Zur Schaumstabilisierung und Viskositätseinstellung werden den Haarshampoos auch Fettsäurealkanolamide zugesetzt. Darüber hinaus können zahlreiche weitere Zusätze, wie Duftstoffe, Konservierungsmittel, Farbstoffe und Trübungsmittel enthalten sein. Durch Zusätze von Ölkomponenten, Rückfettungsmitteln, Lösungsvermittlern, Verdickungsmitteln oder Wirkstoffen gegen Schuppen und gegen das Nachfetten der Haare ist eine weitere Spezialisierung der erfindungsgemässen Haarshampoos möglich.

Die weiteren erfindungsgemäss durch Zusätze von kolloidalen Lösungen von Seidenfibroin zu verbessernden haarkosmetischen Mittel enthalten neben 0,01 bis 1,0 Gewichtsprozent der kolloidal gelösten Seide und den zur Auflösung der Seide erforderlichen Menge an Salzen

zur Verwendung als Haarnachspülmittel, z.B. als Haarkur, vorwiegend Cetyl- und Stearylalkohol und Fettsäurepartialglyceride, z.B. Glycerinmono-di-stearat als Verdickungsmittel quartäre Ammoniumverbindungen als Avivagewirkstoffe, Cellulosederivate, z.B. Hydroxyethylcellulose als Filmbildner sowie Parfümöle, Lösevermittler, Farbstoffe und Wasser,

zur Verwendung als Haarwellmittel, z.B. als Dauerwellmittel vorwiegend Reduktionsmittel für das Haarkeratin, z.B. alkalische Mercaptane wie Ammoniumthioglykolat oder Alkalisulfit, Komplexbildner wie z.B. Hydroxyethandiphosphonsäure, Emulgatoren wie z.B. Fettalkoholpolyglykolether oder Fettalkoholsulfat sowie Parfümöle, Farbstoffe und Wasser,

zur Verwendung als Haarfärbemittel, z.B. als Haarfärbecreme, eine verdickende Emulsionsbasis, bestehend z.B. aus Fettalkoholen und Fettalkoholsulfaten oder Fettalkoholpolyglykolethersulfaten, bevorzugt als Lithiumsalze, einen oder mehrere Oxidationshaarfarbstoffe vom Entwickler-Typ, einen oder mehrere Oxidationshaarfarbstoffe vom Kuppler-Type, ein Reduktionsmittel zur Stabilisierung, z.B. Natriumsulfit oder Thioglykolat sowie Ammoniak und Wasser.

Die nachfolgenden Beispiele sollen den Gegenstand der Erfindung näher erläutern ohne ihn jedoch hierauf zu beschränken.

Beispiele
1. Entbastung von Naturseide.

1.1 Unbehandelte Kokons werden mit einer Schere aufgeschnitten und die Puppen entfernt. In eine Lösung von 375 g Marseiller Seife und 7125 g Wasser von 60 °C werden 300 g der zerschnittenen, von den Puppen befreiten Kokons gegeben. Das Gemisch wird zum Sieden erhitzt und 5 Stunden gekocht. Dann wird die wässrige Seifenlösung dekantiert und die Seide viermal mit heissem Wasser nachgewaschen. Danach wird die Seide durch Schleudern vom Wasser befreit und getrocknet.

1.2 Unbehandelte Kokons werden in einer Mühle zerschnitten und die Puppen soweit wie möglich entfernt. In eine Lösung von 210 g Marseiller Seife in 18 kg Wasser von 60 °C werden 625 g der zerschnittenen, von den Puppen befreiten Kokons gegeben. Das Gemisch wird zum Sieden erhitzt und 5 Stunden gekocht. Dann wird die Seifenlösung abgetrennt und die entbastete Seide mit heissem Wasser viermal gewaschen. Danach wird die gewaschene Seide trockengeschleudert.

2. Herstellung der kolloidalen Lösung von Seidenfibroin.

2.1 In eine Lösung von 870 g Lithiumbromid in 840 g Wasser werden 190 g der nach Beispiel 1.1 entbasteten Seide gegeben. Nach

48 Stunden hat sich die Seide bei Raumtemperatur von 25 °C hat sich die Seide bei Raumtemperatur von 25 °C vollständig gelöst. Die klare, schwach bräunliche Lösung hat eine Viskosität von 5 Pa.s bei 25 °C und enthält 10 Gewichtsprozent kolloidal gelöstes Seidenfibroin.

2.2 In eine Lösung von 460 g Lithiumbromid in 440 g Wasser werden 100 g der nach Beispiel 1.2 entbasteten Seide gegeben. Nach 48 Stunden ist die Seide fast völlig gelöst. Von Spuren ungelösten Materials wird abfiltriert. Die klare, schwach bräunliche Lösung hat eine Viskosität von 7,6 Pa.s bei 25 °C und enthält 10 Gewichtsprozent kolloidal gelöstes Seidenfibroin.

3. Herstellung von haarkosmetischen Mitteln. Nachfolgend werden einige Rezepturen von haarkosmetischen Mitteln mit einem Gehalt an kolloidal gelöstem Seidenfibroin aufgeführt:

3.1 Haarshampoos

| Bestandteile | A | B | C | D | E | F |
|---|---|---|---|---|---|---|
| Fettalkohol $C_{12-14}$-polyglykolethersulfat, Na-Salz (2 EO) | 5 | – | – | 5 | – | – |
| Fettalkohol $C_{12-18}$-polyglykolethersulfat, Mg-Salz (6 EO) | – | 5 | – | – | 5 | – |
| Fettalkohol $C_{12-14}$-polyglykolethersulfat, $NH_4$-Salz (2 EO) | – | – | 5 | – | – | 5 |
| Fettalkohol $C_{12/14}$-sulfat, Li-Salz | 10 | 10 | 10 | 10 | 10 | 10 |
| Kokosfettsäurediethanolamid | | | | | | |
| Fettalkohol $C_{12-18}$-polyglykolether (10 EO) | 2 | 2 | 2 | 2 | 2 | 2 |
| Fettsäure $C_{10-18}$amidopropyldimethylaminoessigsäure-Betain | 3 | 3 | 3 | – | – | – |
| Fettsäure $C_{10-18}$-amidopropyldimethylaminoxid | – | – | – | 3 | 3 | 3 |
| Wässrige Lösung von 10 Gew.-% Seidenfibroin nach 2.1 | 5 | 5 | 5 | 5 | 5 | 5 |
| Wasser, Farbstoff, Parfüm | 75 | 75 | 75 | 75 | 75 | 75 |

Die erhaltenen Shampooformulierungen sind klare, stabile flüssig-viskose Lösungen.

3.2 Haarkur

| | G |
|---|---|
| Fettalkohol $C_{16-18}$ | 2 |
| Glycerin-mono-distearat | 2 |
| Cetyltrimethylammoniumchlorid | 0,5 |
| Polyoxypropylen-methyl-diethyl-ammoniumchlorid | 0,3 |
| Hydroxyethylcellulose | 0,5 |
| Wässrige Lösung von 10 Gew.-% Seidenfibroin nach 2.1 | 2,1 |
| Wasser, Farbstoff, Parfüm | 92,6 |

Das erhaltene Produkt ist eine stabile, flüssige Emulsion.

3.3 Dauerwellmittel

| | H |
|---|---|
| Thioglykolsäure | 7,5 |
| Ammoniak-Lösung (25 gew.-%ig) | 8,0 |
| Hydroxyethandiphosphonsäure | 0,5 |
| Fettalkohol $C_{12/14}$-sulfat, Li-Salz | 3,0 |
| Wässrige Lösung von 10 Gew.-% Seidenfibroin nach 2.1 | 2,0 |
| Wasser, Farbstoff, Parfüm | 79,0 |

Schwach opake, dünnflüssige Lösung

### 3.4 Haarfärbemittel

| | I |
|---|---|
| Fettalkohol C$_{12-18}$ | 8 |
| Fettalkohol C$_{12-14}$-Sulfat, Li-Salz | 12 |
| 2.4.5.6-Tetraaminopyrimidin-sulfat-Monohydrat | 0,3 |
| 2-Methylresorcin | 0,075 |
| 2.7-Dihydroxynaphthalin | 0,075 |
| m-Aminophenol | 0,01 |
| Resorcin | 0,05 |
| p-Toluylendiamin-sulfat | 0,10 |
| p-Aminophenolhydrochlorid | 0,15 |
| Ethylendiaminotetraessigsäure | 0,2 |
| Thioglykolsäure | 0,2 |
| Ammoniak-Lösung, 25 gew.-%ig | 6,0 |
| Wässrige Lösung von 10 Gew.-% Seidenfibroin nach 2.1 | 4,0 |
| Wasser | 68,83 |

Haarfärbecreme, geeignet für Tubenabfüllung, zur Erzeugung einer goldblonden Haarfärbung.

**Patentansprüche**

1. Verwendung kolloidaler Lösungen von Seidenfibroin in konzentrierten wässrigen Lösungen der Chloride und/oder Bromide des Lithiums und/oder Magnesiums als haarpflegende Zusätze zu haarkosmetischen Mitteln.

2. Verwendung kolloidaler Lösungen von Seidenfibroin nach Anspruch 1, dadurch gekennzeichnet, dass eine bei Temperaturen unterhalb +60 °C hergestellte Lösung von Seidenfibroin in einer wässrigen konzentrierten Lösung von Lithiumbromid verwendet wird.

3. Verwendung kolloidaler Lösungen von Seidenfibroin nach Anspruch 1 oder 2, dadurch gekennzeichnet, dass die Lösungen in solchen Mengen zugesetzt werden, dass die haarkosmetischen Mittel 0,01 bis 1,0 Gewichtsprozent an kolloidal gelöster Seide enthalten.

4. Haarshampoo, dadurch gekennzeichnet, dass es 0,1 bis 1,0 Gewichtsprozent kolloidal gelöster Seide, die zur Auflösung der Seide erforderliche Menge an Chloriden und/oder Bromiden des Lithiums und/oder Magnesiums, 5 bis 20 Gewichtsprozent anionischer Sulfattenside wie Fettalkoholsulfate und Fettalkoholpolyglykolethersulfate mit 2 bis 10 Glykolethergruppen im Molekül, oder Sulfonattenside wie sekundäre Alkansulfonate und α-Olefinsulfonate mit 10 bis 20 Kohlenstoffatomen im Molekül, in Form der Ammonium, Alkanolammonium, Alkali- oder Magnesiumsalze, bevorzugt in Form der Lithiumsalze, sowie gegebenenfalls nichtionische und ampholytische Tenside, Fettsäurealkanolamide und weitere übliche Shampoobestandteile enthält.

**Claims**

1. The use of colloidal solutions of silk fibroin in concentrated aqueous solutions of the chlorides and/or bromides of lithium and/or magnesium as hair-care additives in hair-cosmetic preparations.

2. The use of colloidal solutions of fibroin as claimed in Claim 1, characterized in that a solution of fibroin prepared at temperatures below +60 °C in an aqueous concentrated solution of lithium bromide is used.

3. The use of colloidal solutions of fibroin as claimed in Claim 1 or 2, characterized in that the solutions are added in such quantities that the hair-cosmetic preparations contain from 0.01 to 1.0% by weight colloidally dissolved silk.

4. A hair shampoo, characterized in that it contains from 0.1 to 1.0% by weight colloidally dissolved silk, chlorides and/or bromides of lithium and/or magnesium in the quantity necessary for dissolving the silk, from 5 zo 20% by weight anionic sulfate surfactants, such as fatty alcohol sulfates and fatty alcohol polyglycol ether sulfates containing from 2 to 10 glycol ether groups in the molecule or sulfonate surfactants, such as secondary alkane sulfonates and α-olefin sulfonates containing from 10 to 20 carbon atoms in the molecule in the form of the ammonium, alkanolammonium, alkali or magnesium salts, preferably in the form of the lithium salts, and optionally nonionic and ampholytic surfactants, fatty acid alkanolamides and other standard shampoo ingredients.

**Revendications**

1. Utilisation de solutions colloïdales de fibroïne de la soie dans des solutions aqueuses concentrées des chlorures et/ou bromures de lithium et/ou de magnésium, comme additifs pour les soins de la chevelure à des agents cosmétiques capillaires.

2. Utilisation de solutions colloïdales de fibroïne de la soie suivant la revendication 1, caractérisée en ce que l'on utilise une solution préparée à des températures inférieures à +60 °C, de fibroïne de la soie dans une solution aqueuse concentrée de bromure de lithium.

3. Utilisation de solutions colloïdales de la fibroïne de la soie suivant les revendications 1 ou 2, caractérisée en ce que les solutions sont réunies dans des proportions telles que les agents cosmétiques capillaires contiennent 0,01 à 1% en poids de soie dissoute colloïdale.

4. Shampoing pour les cheveux, caractérisé en ce qu'il contient 0.1 à 1% en poids de soie en solution colloïdale, la quantité de chlorure et/ou de bromure de lithium et/ou de magnésium nécessaire pour la dissolution de la soie, 5 à 20% en poids de sulfate tensio-actif anionique, tel qu'un sulfate d'alcool gras et un polyglycol éthersulfate d'alcool gras avec 2 à 10 groupes glycoléther dans la molécule, ou des sulfonates tensio-actifs, tels que des sulfonates d'alcanes secondaires et des sulfonates d'α-oléfines avec 10 à 20 atomes de carbone dans la molécule, sous la forme de sels d'ammonium, d'alcanolammonium, de métaux alcalins ou de sels de magnésium, de préférence sous la forme de sels de lithium, ainsi qu'éventuellement des tensio-actifs non-ioniques et ampholytiques, des alcanolamides d'acides gras et autres parties constituantes habituelles des shampoings.